# EUROPEAN PATENT APPLICATION

(11) **EP 2 444 493 A1**
(43) Date of publication of application: **25.04.2012**
(21) Application number: 10789569.0
(22) Date of filing: 18.06.2010
(51) Int. Cl.: C12N 15/09, A01H 1/00, A01H 5/00, C12N 1/15, C12N 1/19, C12N 1/21, C12N 5/10, C12N 9/10, C12Q 1/68

(54) **PROTEIN HAVING NOVEL PRENYLATION ENZYME ACTIVITY AND GENE ENCODING SAME**

(30) Priority: 19.06.2009 JP 2009146754
(71) Applicant: Kirin Holdings Kabushiki Kaisha, Tokyo 104-8288 (JP)
(72) Inventor: YAZAKI Kazufumi, Uji-shi Kyoto 611-0011 (JP); UMEMOTO Naoyuki, Sakura-shi Tochigi 329-1414 (JP); MOMOSE Masaki, Sakura-shi Tochigi 329-1414 (JP)
(74) Representative: HOFFMANN EITLE
(86) International application number: PCT/JP2010/060328
(87) International publication number: WO 2010/147196

(57) **Abstract**

This invention provides DNAs of a prenyltransferase derived from a hop (*Humulus*) plant. The invention also provides protein having the prenyltransferase activity of a hop plant and a method for producing and detecting a novel organism using a gene encoding such protein.

## Description

### Technical Field

The present invention relates to a method for producing characteristic prenylated compounds of hop, prenyltransferase, DNA encoding such enzyme, and methods of breeding and selecting a novel hop plant using such DNA.

### Background Art

The term "prenylation" refers to a reaction in which a hydrophobic prenyl group is added to a compound. A prenyl group significantly influences the structure of plant secondary metabolites and the diversity of physiological activities, and a prenylated aromatic compound serves as a major resource for naturally occurring organic compounds. Such compounds are biosynthesized via a pathway referred to as a "complex pathway" from a biosynthetic point of view, and they play a key role in plant life maintenance in terms of, for example, insect resistance or disease resistance. Some prenylated aromatic compounds contribute to pharmacological actions of medicinal plants as physiologically active substances (Non-Patent Document 1). For example, prenylated flavonoids are reported to have a variety of types of physiological activity, such as antitumor activity and antibacterial activity (Non-Patent Document 2), and these compounds are recognized as very important in the pharmaceutical and food industries. These compounds play a key role in protecting plants from insect damage or infection.

It has been reported that a prenyl group is important for physiological activity of a prenylated aromatic compound, such as a prenylated flavonoid. In contrast to a prenylated aromatic compound having physiological activity, many mother compounds that have no prenyl group have no physiological activity. An enzyme that catalyzes prenylation of an aromatic compound is very important at the industrial level since it is capable of potentiating the physiological activity of an aromatic compound. However, many prenyltransferases having aromatic substrates are membrane-bound enzymes, and analysis thereof has not been easy. Regarding a plant prenyltransferase having a flavonoid substrate, in particular, cloning of a gene derived from a medicinal plant (i.e., *Sophora flavescens*) was recently reported for the first time ever (Patent Document 1).

Prenylated compounds are particularly important for hop (*Humulus*). Hop, which is an important raw material of beer, makes beer bitter. Hop is known to be composed of a variety of bitter acid ingredients, and beer taste varies depending on various compositions of such ingredients (Non-Patent Document 3). Representatives of hop ingredients include α acid (humulone) and β acid (lupulone), which are special prenylated compounds. Meanwhile, prenylated flavonoids are known to exhibit very interesting physiological activity (Non-Patent Document 4). For example, xanthohumol exhibits extensive antitumor activity. 8-Prenylnaringenin exhibits the most potent estrogenic activity. Such substances are produced in lupulin glands in the hop cone. Reports regarding prenyltransferase activity are limited to activity about bitter acids and the activity was reported to be present in a soluble fraction (Non-Patent Document 5). There have been no reports regarding prenylated flavonoid. In addition, there has been no research regarding the genes.

In recent years, a low-molecular-weight prenyl synthase (i.e., geranyl(geranyl) diphosphate synthase) has been demonstrated to exhibit geranylgeranyl activity in the form of a homodimer and to function as a geranyl diphosphate synthase in the form of a heterodimer. Regarding geranylgeranylation and geranylation activities, activity ratios may vary depending on association with various modifying factors. However, a distinctive feature of the aforementioned activity relates to a prenyl synthase (Non-Patent Document 6).

### Prior Art Documents

### Patent Document

Patent Document 1: JP Patent Publication (Kokai) No. 2008-220304 A

### Non-Patent Documents

Non-Patent Document 1: Mesia-Vela et al., Phytomedicine 8, 2001, pp. 481-488
Non-Patent Document 2: Sohn et al., Phytomedicine 11, 2004, pp. 666-672
Non-Patent Document 3: Pollock (ed.) Brewing Science (Food Science & Technological Monograph) Academic Press, 1979
Non-Patent Document 4: Stevens and Page, Phytochemistry, 2004, 65:1317-30
Non-Patent Document 5: Zuurbier et al., Phytochemistry, 1998, 49: 2315-2322
Non-Patent Document 6: Tholl et al., Plant Cell, 2004, 16: 977-992

### Summary of the Invention

### Object to Be Attained by the Invention

It is an object of the present invention to provide a protein having the novel prenyltransferase activity of hops, a gene encoding such protein, and a method for producing and testing a novel organism using the gene encoding such protein.

### Means for Attaining the Object

The present inventors have conducted concentrated studies in order to attain the above object. As a result, they identified prenyltransferase genes, which are different from a bitter acid enzyme deduced to be present in a soluble fraction and have not yet been found in hop. In addition, they demonstrated that expression of such gene would enable production of novel prenylated compounds, and comparison of the genome sequences of such gene among various types of hop plants would enable polymorphism analysis, thereby enabling a newly-bred variety of a hop plant to be demonstrated. This has led to the completion of the present invention.

Specifically, the present invention includes the following.
[1] A protein (a) or (b) below:
   (a) a protein comprising the amino acid sequence as shown in SEQ ID NO: 1; or
   (b) a protein comprising an amino acid sequence derived from the amino acid sequence as shown in SEQ ID NO: 1 by deletion, substitution, insertion, or addition of 1 or several amino acids and having prenyltransferase activity.
[2] A gene comprising any of DNAs (c) to (f) below:
   (c) DNA comprising the nucleotide sequence as shown in SEQ ID NO: 3;
   (d) DNA hybridizing under stringent conditions to DNA comprising a nucleotide sequence complementary to DNA comprising the nucleotide sequence as shown in SEQ ID NO: 3 and encoding a protein having prenyltransferase activity;
   (e) DNA comprising a nucleotide sequence having 80% or higher sequence identity with the nucleotide sequence as shown in SEQ ID NO: 3 and encoding a protein having prenyltransferase activity; and
   (f) DNA comprising a degenerate variant of the nucleotide sequence as shown in SEQ ID NO: 3.
[3] A protein (g) or (h) below:
   (g) a protein comprising the amino acid sequence as shown in SEQ ID NO: 2; or
   (h) a protein comprising an amino acid sequence derived from the amino acid sequence as shown in SEQ ID NO: 2 by deletion, substitution, insertion, or addition of 1 or several amino acids and having prenyltransferase activity.
[4] A gene comprising any of DNA (i) to (1) below:
   (i) DNA comprising the nucleotide sequence as shown in SEQ ID NO: 4;
   (j) DNA hybridizing under stringent conditions to DNA comprising a nucleotide sequence complementary to DNA comprising the nucleotide sequence as shown in SEQ ID NO: 4 and encoding a protein having prenyltransferase activity;
   (k) DNA comprising a nucleotide sequence having 80% or higher homology to the nucleotide sequence as shown in SEQ ID NO: 4 and encoding a protein having prenyltransferase activity; and
   (l) DNA comprising a degenerate variant of the nucleotide sequence as shown in SEQ ID NO: 4.
[5] A recombinant vector comprising the gene according to [2] or [4].
[6] A transformant into which the recombinant vector according to [5] has been introduced.
[7] The transformant according to [6], which is a plant.
[8] A method for detecting the presence of mutation and/or polymorphism of a gene encoding prenyltransferase in a plant comprising steps of:
   (i) isolating a nucleic acid, which is genomic DNA or RNA, from a plant;
   (ii) synthesizing cDNA via reverse transcription when the nucleic acid of (i) is RNA;
   (iii) amplifying a gene fragment comprising the nucleotide sequence as shown in SEQ ID NO: 3, SEQ ID NO: 4, or SEQ ID NO: 11 from DNA obtained by step (i) or (ii); and
   (iv) determining the presence of mutation and/or polymorphism in DNA.
[9] The method according to [8], wherein the plant is of the genus *Humulus.*
[10] A method for selecting a plant comprising mutation and/or polymorphism by detecting mutation and/or polymorphism of a gene encoding a prenyltransferase by the method according to [8] or [9].
[11] A plant selected by the method according to [9] having mutation and/or polymorphism of a gene encoding a prenyltransferase.
[12] The plant according to [11], which is of the genus *Humulus.*
[13] The method for selecting a plant according to [8] or [9] comprising selecting a plant having the capacity for expressing a gene encoding a prenyltransferase or activity of a prenyltransferase to be encoded that is altered from that of an existing plant variety.
[14] A plant selected by the method according to [13] having the capacity for expressing a gene encoding a prenyltransferase or activity of a prenyltransferase that is altered from that of an existing plant variety.
[15] The plant according to [14], which is of the genus *Humulus.*

This description includes part or all of the contents as disclosed in the description and/or drawings of Japanese Patent Application No. 2009-146765, which is a priority document of the present application.

### Effects of the Invention

According to the present invention, expression of the activity of a protein having novel prenyltransferase activity originating from hops and of a gene encoding such protein can be regulated. That is, the present invention provides a method for producing a plant in which the activity of such gene is regulated. The present invention enables breeding of hops characterized by compositions of prenylated compounds. With the use of the enzyme of the present invention, large quantities of prenyl aromatic compounds exhibiting a variety of useful physiological activities can be produced in a cost-effective manner.

### Brief Description of the Drawings

Fig. 1A shows the results of homology searches of a partial H1PT1 sequence (1,211 nucleotides) and the plastoquinone synthase gene having a solanesyl diphosphate substrate by BLASTX using the database: NCBI/blast/db/FASTA/2008_09_05_09_00_0/nr 6,937,173 sequences; 2,395,280,820 total letters (summary). Underlined regions indicate homologous regions, but such homology is limited and partial.
Fig. 1B is a continuation from Fig. 1A.
Fig. 2A shows the results of homology searches of a partial H1PT2 sequence (693 nucleotides) and the plastoquinone synthase gene having a solanesyl diphosphate substrate by BLASTX using the database: NCBI/blast/db/FASTA/2008_09_05_09_00_0/nr 6,937,173 sequences; 2,395,280,820 total letters (summary). Underlined regions indicate homologous regions, but such homology is limited and partial.
Fig. 2B is a continuation from Fig. 2A.
Fig. 3 shows the result of analysis of the molecular evolutionary phylogenetic trees of H1PT1 and H1PT2.
Fig. 4 shows geranylnaringenin production via H1PT1 and H1PT2 gene expression.
   It shows generation of at least 3 products (i.e., products a, b, and c). Product c was identified as 6-geranylnaringenin based on Fig. 5.
Fig. 5 shows the results of identification of 6-geranylnaringenin.
Fig. 6 shows geranylisoliquiritigenin production via H1PT1 gene expression.
Fig. 7 shows production of geranylsakuranetin, geranyltaxifolin, and geranylgenistein via H1PT1 gene expression.
Fig. 8 shows the results of identification of xanthoangelol.
Fig. 9 shows the results of analysis of H1PT1 and H1PT2 expression in "Kirin II" and "Toyomidori" leaves. Expression levels of both genes were found to be higher in "Toyomidori" than in "Kirin II."

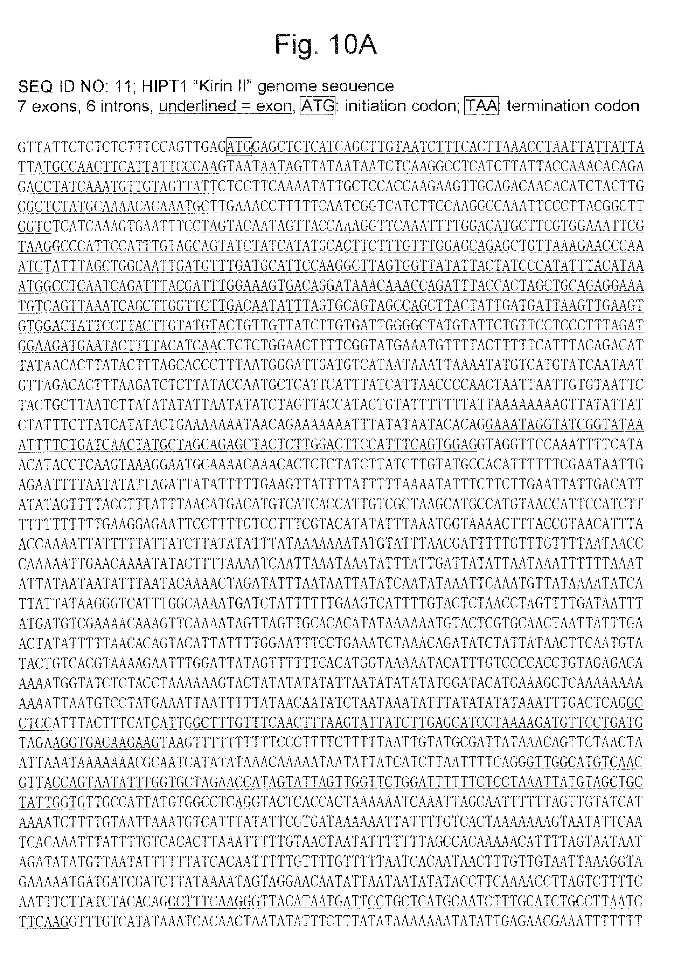

Fig. 10A shows the genome sequence of "Kirin II."

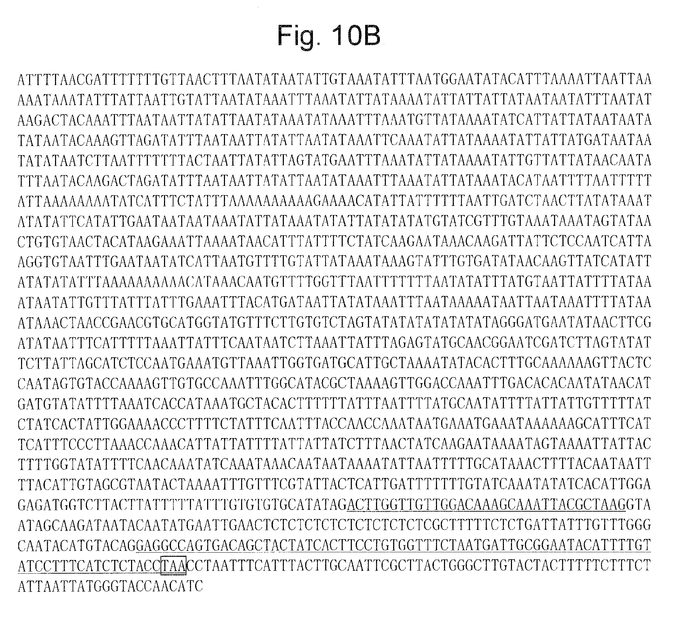

Fig. 10B shows the genome sequence of "Kirin II" (continued from Fig. 10A).

### Embodiments for Carrying out the Invention

Hereafter, the present invention is described in detail.

### 1. Novel prenyltransferase

The proteins of the present invention are prenyltransferase having extensive activity of binding a prenyl group to an aromatic compound. The enzyme of the present invention is a prenyltransferase derived from a plant of the genus *Humulus* (*Humulus lupulus*), such as hops (*Humulus lupulus*). Examples of plants of the genus *Humulus* include *Humulus lupulus, Humulus lupulus var. cordifolius,* and *Humulus japonicus.* Further, the enzyme of the present invention is a membrane-bound prenyltransferase.

Examples of preferable aromatic compounds serving as the prenyltransferase substrates in the present invention include flavonoid, isoflavonoid, coumarin, chalcone, and phloroglucinol. Examples of flavonoids include naringenin, hesperetin, galangin, chrysin, isosakuranetin, isoliquiritigenin, sakuranetin, taxifolin, genistein, and 2',4',4-trihydroxy-6'-methoxychalcone. Examples of phloroglucinol include phlorisovalerophenone (PIVP), phlorisobutyrophenone (PIBP), and phlormethylbutanophenone (PMBP). The prenyltransferase of the present invention binds a prenyl group having an isoprene unit containing 5 carbon atoms to an aromatic compound. Examples of prenyl groups include a dimethylallyl group (5 carbon atoms), a geranyl group (10 carbon atoms), a farnesyl group (15 carbon atoms), and a geranylgeranyl group (20 carbon atoms). Examples of prenyl group donors serving as substrates that donate prenyl groups include dimethylallyl diphosphate (DMAPP), geranyl diphosphate (GPP), farnesyl diphosphate (FPP), geranylgeranyl diphosphate (GGPP), and phytyl diphosphate (PDP). A prenyl group is transferred from such prenyl group donor to the aromatic compound by the catalytic action of the prenyltransferase.

The full-length amino acid sequences of the enzyme of the present invention are shown in SEQ ID NO: 1 and 2. Further, the protein of the present invention includes a protein having an amino acid sequence that is substantially the same as the amino acid sequence as shown in SEQ ID NO: 1 or 2 and having prenyltransferase activity. Examples of such substantially similar amino acid sequences include an amino acid sequence derived from the amino acid sequence of interest by deletion, substitution, insertion, and/or addition of 1 or a plurality of or several amino acids (e.g., 1 to 10, preferably 1 to 7, more preferably 1 to 5, further preferably 1 to 3, and still further preferably 1 or 2 amino acids) and an amino acid sequence having at least 85%, preferably 90% or higher, more preferably 95% or higher, and particularly preferably 97% or higher sequence identity with the amino acid sequence of interest, when calculated using, for example, BLAST (i.e., the basic local alignment search tool at the National Center for Biological Information, U.S.A.) (e.g., with the use of default; i.e., initial parameters).

The prenyltransferase of the present invention encompasses a naturally occurring prenyltransferase isolated from a plant and a recombinant prenyltransferase produced via genetic engineering.

### 2. Gene encoding prenyltransferase

The gene of the present invention encodes a prenyltransferase having activity of binding a prenyl group to an aromatic compound and encodes a protein having the prenyltransferase activity described above.

The nucleotide sequences of DNA of the gene of the present invention are shown in SEQ ID NO: 3 and 4. Further, DNA of the present invention encompasses DNA hybridizing under stringent conditions to the DNA having a nucleotide sequence complementary to the nucleotide sequence as shown in SEQ ID NO: 3 or 4, DNA having at least 85%, preferably 90% or higher, more preferably 95% or higher, and particularly preferably 97% or higher sequence identity with the nucleotide sequence as shown in SEQ ID NO: 3 or 4, when calculated using, for example, BLAST (i.e., the basic local alignment search tool at the National Center for Biological Information, U.S.A.) (e.g., with the use of default; i.e., initial parameters), or DNA encoding an amino acid sequence derived from the amino acid sequence of a protein encoded by the aforementioned DNA by deletion, substitution, insertion, and/or addition of 1 or a plurality of or several amino acids (e.g., 1 to 10, preferably 1 to 7, more preferably 1 to 5, further preferably 1 to 3, and still further preferably 1 or 2 amino acids), which encodes a protein having prenyltransferase activity. Under "stringent conditions," for example, hybridization is carried out in approximately 1x SSC and 0.1% SDS at 37°C. Under more stringent conditions, hybridization is carried out in approximately 0.5x SSC and 0.1% SDS at 42°C. Under further stringent conditions, hybridization is carried out in approximately 0.2x SSC and 0.1% SDS at 65°C. In addition, the gene of the present invention includes DNA comprising a degenerate variant of the nucleotide sequence as shown in SEQ ID NO: 3 or 4. 3. Recombinant vector

The vector of the present invention is a recombinant vector resulting from insertion of DNA having the sequence as shown in SEQ ID NO: 3 or 4. A wide variety of known vectors for yeast, plant cells, insect cells, and other substances can be used. Examples of known vectors for yeast include pDR196, pYES-DEST 52, Yip5, Yrp17, and Yep24 vectors. Examples of known vectors for plant cells include pGWB, pBiE12-GUS, pIG121-Hm, pBI121, pBiHyg-HSE, pB119, pB1101, pGV3850, and pABH-Hm1 vectors. Examples of known vectors for insect cells include pBM030, pBM034, and pBK283 vectors. The vectors used in the present invention comprise constituents associated with gene expression or suppression, such as promoters, terminators, and enhancers, incorporated therein. The vectors comprise selection markers (e.g., drug resistant genes, antibiotic resistant genes, or reporter genes), according to need. Preferably, constituents associated with gene expression or suppression are functionally incorporated into recombinant vectors in accordance with properties thereof. A person skilled in the art would be able to adequately implement such procedures.

### 4. Transformant

The transformant of the present invention carries the recombinant vector of the present invention. The transformant can be obtained by introducing a recombinant vector comprising an enzyme-encoding gene inserted therein into a host, so that the target gene can be expressed therein. An adequate host for a vector may be used. Examples thereof include yeast, plant cell, insect cell (e.g., Sf9), and plant virus hosts. Yeast, plant cell, or plant virus hosts are preferable, for example. A recombinant vector can be introduced into a microbial host by any method without particular limitation, provided that the method allows introduction of DNA into a microbial host. Examples include a method involving the use of calcium ions (Cohen, S. N.et al., Proc. Natl. Acad. Sci., U.S.A., 69: 2110, 1972), electroporation, and tri-parental mating. An example of a method for producing a transformed plant is a method using a virus, Agrobacterium Ti-plasmid, or Agrobacterium Ri plasmid as a vector. Examples of host plants include monocotyledons, such as rice, wheat, or maize, and dicotyledons, such as soybean, rapeseed, tomato, or potato. The transformed plant can be obtained by regenerating from a plant cell transformed with the gene of the present invention. Plant regeneration from a plant cell can be carried out by a known method.

### 5. Method for producing prenyltransferase

The prenyltransferase of the present invention can be collected from a common plant or the transformant mentioned above. Specifically, the method of Yazaki et al (JBC, 2002, 277,6240-6246) can be employed, for example.

In addition, the prenyltransferase of the present invention can be mass-produced using a microbial expression system, such as yeast transformed with the gene of the present invention, for example. Also, the prenyltransferase can be obtained by expressing the gene of the present invention in the transformed plant mentioned above.

Transformants may be cultured using a known medium by a known method.

### 6. Production of prenylated aromatic compound using transformant

The membrane-bound prenyltransferase of the present invention can be expressed in the form of a protein that is highly active in a microorganism such as yeast. Thus, the prenyltransferase substrate may be added to the culture solution of transformed yeast to produce a prenylated aromatic compound. For example, a flavonoid, such as naringenin, may be introduced as a substrate into a culture solution of transformed yeast to effectively mass-produce prenylated flavonoids. Since naringenin has adequate degrees of water solubility and hydrophobic properties, it penetrates a biomembrane and enters into a yeast cell, and it then comes into contact with a prenyltransferase in a yeast cell. Yeast has a pathway for biosynthesizing DMAPP in the cytosol (i.e., the mevalonate pathway), and a prenyl substrate as a prenyl group donor is supplied *in vivo.* Produced prenylated flavonoids can be obtained from a culture product of yeast. The term "culture product of yeast" used herein refers to a yeast cell and/or medium.

Also, the membrane-bound prenyltransferase of the present invention may be expressed in a plant cell or plant transformed with the gene of the present invention, so that a prenylated aromatic compound, such as a prenylated flavonoid, can be produced in a plant. In the case of a plant cell, there are two pathways for DMAPP biosynthesis (i.e., the mevalonate pathway in the cytosol and the non-mevalonate pathway localized in the plastid). In order to produce a prenylated aromatic compound using DMAPP synthesized in the former pathway as a prenyl group donor, a modified gene from which the plastid localization signal has been removed may be used. In order to produce a prenylated aromatic compound using DMAPP synthesized in the latter pathway as a prenyl group donor, the endogenous plastid localization signal may be used, or a plastid localization signal of another gene, such as the RuBisCo small subunit, may be ligated to localize a prenyltransferase in the plastid. In the case of a flavonoid aromatic compound, such as a flavonoid substrate, an endogenous aromatic compound is prenylated. Thus, a prenylated aromatic compound can be recovered from plant tissue of a grown plant. Alternatively, a plant is allowed to absorb a flavonoid, such as naringenin, for prenylation, and prenylated flavonoid can be recovered from plant tissue. In such a case, flavonoid may be added to, for example, soil to allow a plant to absorb flavonoid through the root. Alternatively, a tissue-specific promoter may be used to produce prenylated flavonoid in arbitrary plant tissue.

As described above, the prenyltransferase and the gene encoding the prenyltransferase of the present invention may be used to prenylate a variety of aromatic compounds, such as flavonoid, to produce a prenylated aromatic compound, such as prenylated flavonoid. Prenylated flavonoid has a variety of types of activity, such as antitumor activity, antibacterial activity, antiviral activity, antioxidative activity, estrogen-like activity, immunological enhancing activity, and anti-inflammatory activity. A compound with enhanced such physiological activities can be produced with the use of the prenyltransferase of the present invention. Also, the hop-derived prenyltransferase of the present invention is associated with synthesis of bitter acids, such as humulone or lupulone. Humulone or lupulone is obtained via prenylation of phlorisovalerophenone (PIVP), phlorisobutyrophenone (PIBP), or phlormethylbutanophenone (PMBP). The prenyltransferase of the present invention may be used in the form of a monomer or dimer.

### 7. Method for measuring activity of the prenyltransferase of the present invention and method for analyzing substrate specificity thereof

Activity of the prenyltransferase of the present invention can be measured by, for example, the method described below.

The enzyme of the present invention (340 µg to 730 µg), a substrate aromatic compound (1 mM) such as flavonoid, a prenyl group donor (1 mM), and MgCl₂ (20 mM) are mixed in a 100 mM Tris-HCl buffer (pH 7.5) to bring the amount of the resultant to 200 µl, and the reaction is allowed to proceed at 30°C overnight. After the completion of the reaction, the product is extracted with ethyl acetate, dried, and dissolved in MeOH. The resultant may be analyzed using a mass spectrometer or via chromatography.

In this case, a variety of substrate aromatic compounds may be used to inspect whether or not each aromatic compound is prenylated. Thus, substrate specificity can be analyzed.

### 8. Selection of gene mutation, polymorphic plant, and mutation in gene expression

The present invention provides a method for detecting the presence of mutation in the prenyltransferase gene, polymorphisms, such as single nucleotide polymorphisms (SNPs), and mutation in gene expression in a plant. Mutation may be caused via radiation, chemical treatment, UV application, or natural mutation.

This method comprises a process of isolating genomic DNA or RNA from mutants or plants of various varieties or cultivated plants, a process of synthesizing cDNA via reverse transcription in the case of RNA, a process of amplifying a gene fragment containing the prenyltransferase gene from the DNA and the cDNA via a DNA amplification technique, and a process of determining the presence of mutation in the DNA. DNA or RNA can be extracted using a commercially available kit (e.g., DNeasy or RNeasy, Qiagen). cDNA can be synthesized using a commercially available kit (e.g., SuperScript First-Strand Synthesis System, Invitrogen). A gene fragment can be amplified via a DNA amplification technique, such as so-called PCR or LAMP. These techniques are based on the use of DNA polymerase in order to amplify a specific DNA sequence (i.e., to increase the copy number) via continuous polymerase reactions. While such reactions can be employed instead of cloning, these techniques require only the information regarding nucleic acid sequences. Primers complementary to part of the DNA sequence to be amplified are designed for the purpose of DNA amplification. Subsequently, such primers are prepared via automated DNA synthesis. Methods of DNA amplification are well-known in the art, and a person skilled in the art would be readily able to implement such methods in accordance with the teaching and instructions provided in this description. Several PCR techniques (and associated techniques) are described in, for example, US Patent Nos. 4,683,195, 4,683,202, 4,800,159, and 4,965,188 and PCR Protocols: A guide to method and applications, edited by Innis et al.

The process of determining the presence of mutations or polymorphisms in DNA may be carried out via nucleotide sequencing (with a genetic analyzer, Applied Biosystems) or by a method of detection utilizing homology between a mutant gene and a normal gene, such as the Tilling method (Till et al., 2003, Genome Res. 13: 524-530) that detects mutants with the use of an enzyme cleaving one of a mismatch pair. It can be carried out by comparing the sequence data obtained by such techniques with the nucleotide sequence defined by SEQ ID NO: 3, SEQ ID NO: 4, or SEQ ID NO: 11 associated with the relevant gene region.

In the process of determining mutation resulting from different mRNA levels, the cDNA may be subjected to quantitative PCR, such as real-time PCR using primers prepared based on the nucleotide sequence defined by SEQ ID NO: 3 or SEQ ID NO: 4 (e.g., LightCycler, Roche Diagnostics). Thereafter, the results of PCR may be compared with the cDNA levels obtained from, for example, the "Kirin II" variety to determine mutation that results from different mRNA levels.

In a particularly preferable embodiment, the method for determining the presence of mutation in the prenyltransferase gene defined above is applied to a material obtained from a plant belonging to genus *Humulus,* such as hop, *Humulus lupulus.*

According to the method of determining mutations and/or polymorphisms above, mutations or polymorphisms in a gene encoding a prenyltransferase can be identified at the nucleotide level. In addition, a plant having mutation in a gene encoding a prenyltransferase can be selected and obtained. The present invention encompasses a thus-obtained plant having mutations or polymorphisms in a gene encoding a prenyltransferase.

Based on the determination of mutations or polymorphisms or different mRNA levels, a plant in which the capacity for expressing a gene encoding a prenyltransferase or prenyltransferase activity is altered can be selected. The term "alteration of a given plant activity" refers to alteration of an existing plant variety contained in the plant species of interest, and wild-type plants are within the scope of such existing plant varieties. The term "an existing plant variety" refers to a variety of all plant varieties that can be obtained when a plant in which a gene encoding a prenyltransferase is altered. The altered plant thereof include plant varieties produced via artificial procedures, such as crossing or genetic engineering, and plants that can be distinguished from naturally occurring wild-type plants in terms of enzyme activities. When altering activity, it is not necessary to alter activity in all existing plant varieties. Modification of a given existing plant variety is sufficient to satisfy the condition of "a plant with altered prenyltransferase activity." The term "plant with altered prenyltransferase activity" also refers to a plant in which activity is altered via natural mutation without artificial procedures. A plant in which the activity is altered via natural mutation can be selected and established as a new plant variety by the method of the present invention. When a given existing plant variety is subjected to mutagenesis to produce a plant with altered prenyltransferase activity, a control plant may be the existing plant variety, which had been subjected to mutagenesis, or other existing plant varieties. When a plant is a hop (*Humulus lupulus*), for example, examples of existing plant varieties include Kirin II and Toyomidori. The term "plant in which the capacity for expressing a gene encoding a prenyltransferase or prenyltransferase activity is altered" used herein refers to a plant with the enhanced or attenuated capacity for expressing a gene encoding a prenyltransferase compared with an existing plant variety or a plant with elevated or lowered prenyltransferase activity compared with an existing plant variety. The present invention encompasses plants with the altered capacity for expressing a gene encoding a prenyltransferase or altered prenyltransferase activity.

Such plants, for example having the potentiated capacity for aromatic compound prenylation, or prenyltransferases derived from such plants can be used for more efficient production of prenylated aromatic compounds.

### Examples

Hereafter, the present invention is described in greater detail with reference to the examples, although the present invention is not limited thereto.

### Example 1: Construction of hop gene expression library and nucleotide sequencing

A region containing large quantities of lupulin glands was recovered from the cone of a hop (*Humulus lupulus*) variety (Kirin II) and grounded using liquid nitrogen. Total RNA was extracted by the cetyltrimethylammonium bromide (CTAB) method (Chang et al., 1993, Plant Mol Biol Rep. 11: 113-116). The obtained total RNA (620 µg) was given to Takara Bio Inc. for the purpose of construction of the cDNA library, analysis of 12,288 ESTs, and cluster analysis. Poly(A)+RNA was isolated using the oligotex-dT super mRNA purification kit (Takara Bio Inc.), and cDNA was prepared with the use of a reverse transcriptase. The cDNA plasmid library was constructed using a yeast expression vector (pDR196). The first strand cDNA was synthesized using an oligo(dT)18 anchor primer containing the XhoI restriction site. After the second strand cDNA was synthesized, a blunt-ended adaptor containing the EcoRI restriction site was ligated to double-stranded DNA, and the resulting fragment was then incorporated into a pDR196 plasmid having a constitutive promoter (PMA1). The constructed library of *Escherichia coli* DH10B was subjected to plasmid extraction and 12,288 nucleotide sequences at the 5' end were determined. As a result of cluster analysis, 1,597 clusters of the gene sequences were found to be included.

### Example 2: Extraction of candidate cDNA clones

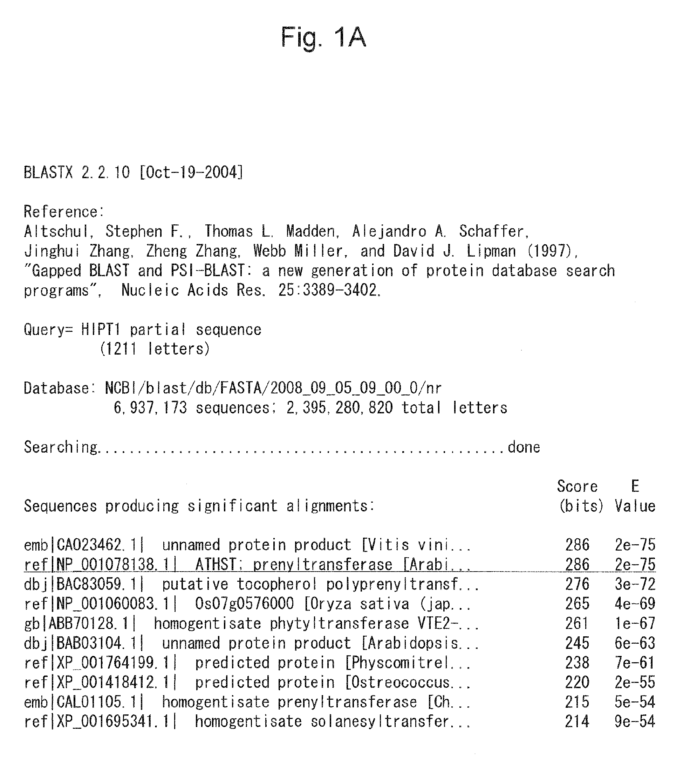

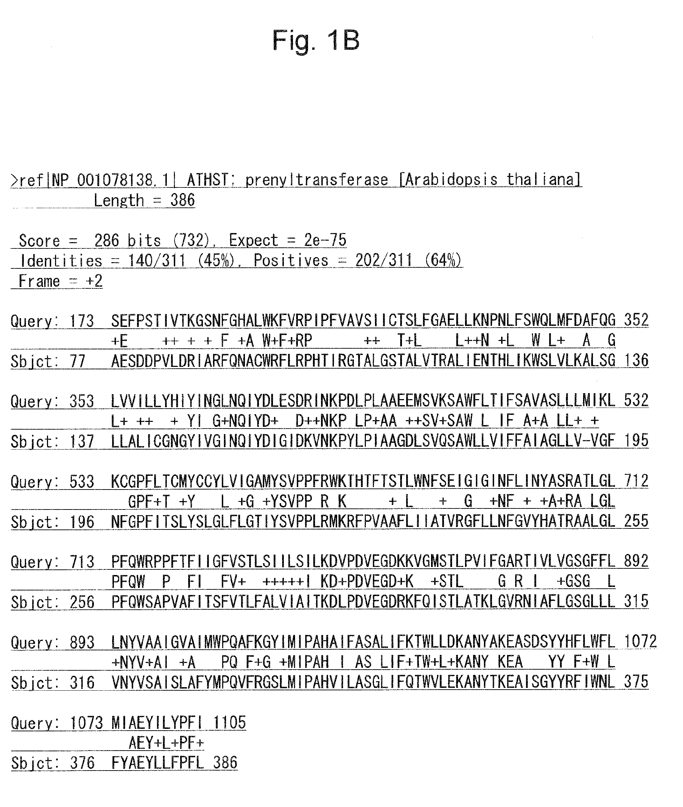

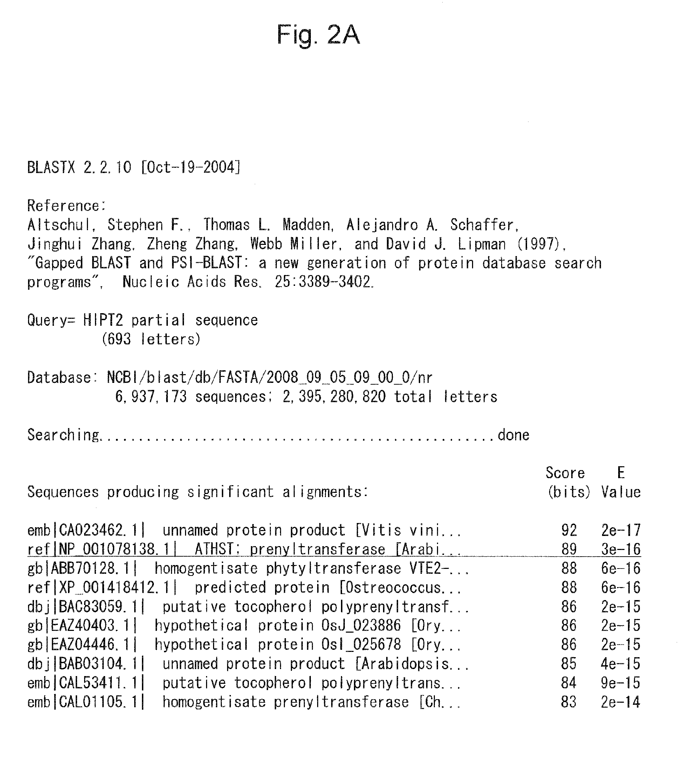

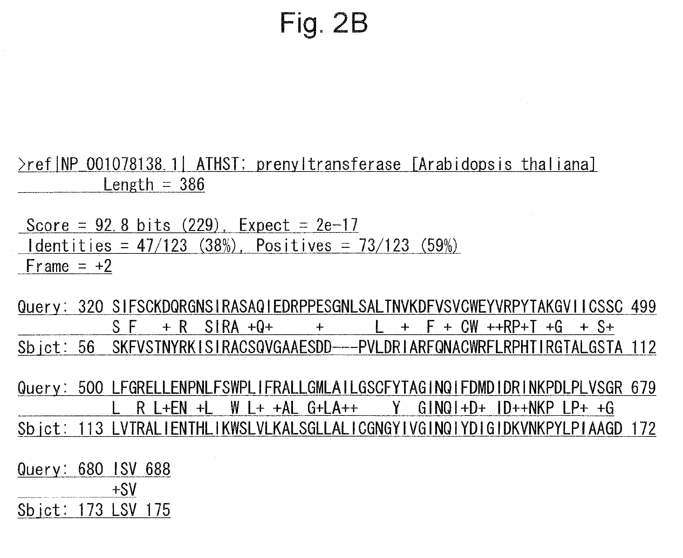

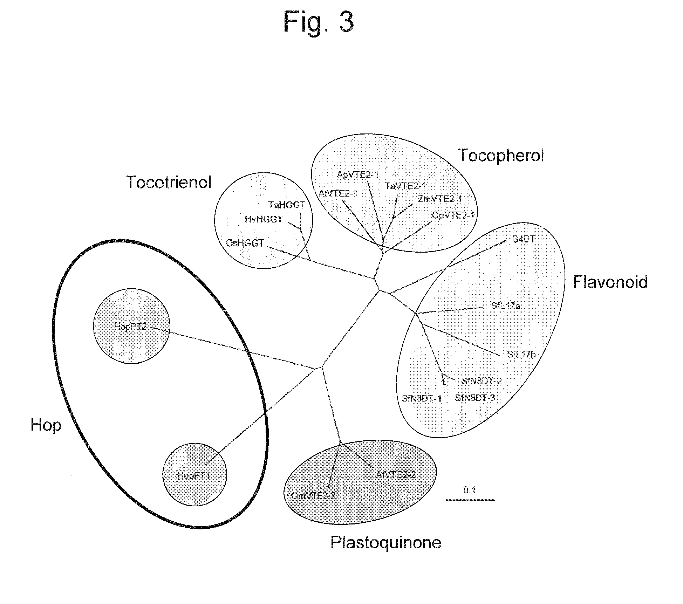

The aforementioned gene clusters were analyzed via nucleotide sequence homology search (BLASTX 2.2.10 (Altschul et al., Nucleic Acids Res. 25: 3389-3402, 1997)) using an amino acid sequence database (Database: NCBI/blast/db/FASTA/2008_09_05_09_00_0/nr., 6,937,173 sequences; 2,395,280,820 total letters) . As a result, two gene clusters (i.e., H1PT1 and H1PT2) were found to be homologous to the plastoquinone synthase gene (Genbank Accession No. ABB701280). Fig. 1A and Fig. 1B each shows data regarding homology between H1PT1 and the plastoquinone synthase gene. Fig. 2A and Fig. 2B each shows data regarding homology between H1PT2 and the plastoquinone synthase gene. Fig. 1B is a continuation from Fig. 1A. Fig. 2B is a continuation from Fig. 2A. In Figs. 1A, 1B, 2A, and 2B, underlined regions exhibit homology. While the sequences showed homology, such homology was partial and limited, and it was impossible to predict the enzyme activity. As is apparent from the examples below, surprisingly, H1PT1 and H1PT2 exhibited activity that is very different from that of the plastoquinone synthase gene. Also, molecular evolutionary phylogenetic trees (ClustalX program: http://www-igbmc.u-strasbg.fr/Biolnfo/ClustalX/Top.html) (Human Genome Sequencing Center, Houston, TX) and TreeView (http://taxonomy.zoology.gla.ac.uk/rod/treeview.html) were drawn with the previously obtained homogentisic acid prenyltransferase gene (tocotrienol or tocopherol) and the prenyl transferase gene for flavonoid including a gene derived from a medicinal plant (*Sophora flavescens*) which had been reported for the first time ever (JP Patent Publication (Kokai) No. 2008-220304 A). As a result, relationships of both H1PT1 and H1PT2 were found to be more distant from above mentioned genes than that of the plastoquinone synthase gene (Fig. 3).

### Example 3: Evaluation of activity of enzyme encoded by candidate cDNAs

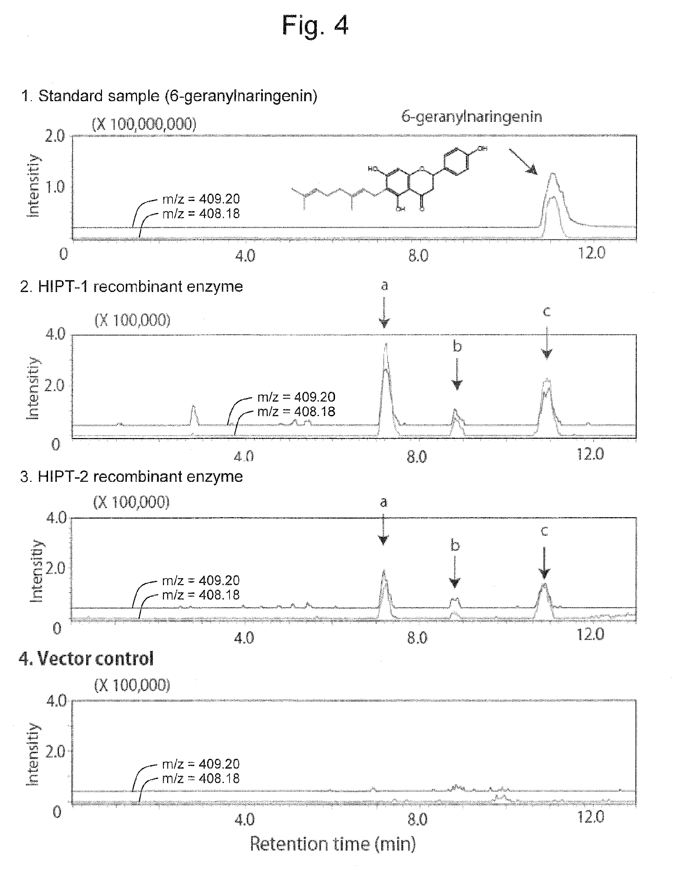

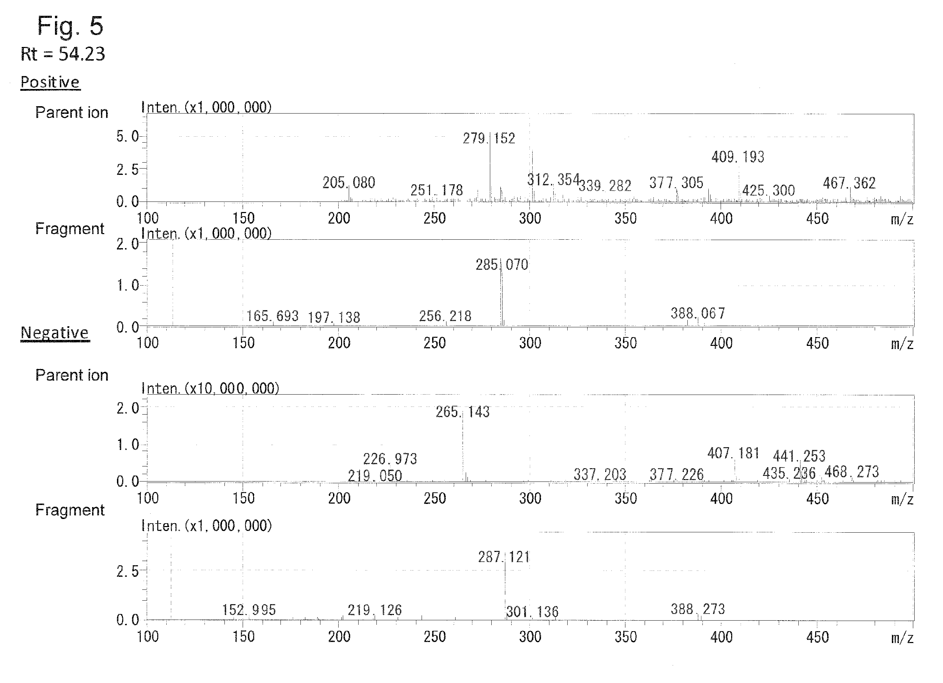

Cloned plasmids (pDR196-H1PT1 and pDR196-H1PT2) comprising each full-length sequence of the two gene clusters (H1PT1 and H1PT2) were introduced into the yeast strain (W303-1A-Δcoq2) by the lithium acetate method. Culture was conducted in 180 ml of SD-Ura liquid medium up to the logarithmic growth phase to express recombinant proteins in yeast transformants, and microsome fractions were prepared therefrom using the method of Yazaki et al. (JBC, 2002, 277, 6240-6246). The total amount of the reaction solution was adjusted to 200 µl by mixing 340 µg to 730 µg of proteins from the microsome fractions, flavonoid (1 mM), a prenyl group donor (1 mM), 20 mM MgCl₂, and 100 mM Tris-HCl buffer (pH 7.5), and the reaction was allowed to proceed at 30°C overnight. After the completion of the reaction, the product was extracted with ethyl acetate, dried, and dissolved in methanol (MeOH). The resultant was analyzed using Shimadzu LCMS-2010A and LCMS-IT-TOF (Shimadzu Corporation), TSK-GEL ODS-80Ts 2.0 mm x 25 cm (TOSOH), and a solvent system (0.3% formic acid:acetonitrile = 3:7) at a flow rate of 0.2 ml/min-1. When naringenin substrate and a prenyl group donor (geranyl diphosphate) were added, a compound into which prenyl had been introduced at position 6 and a compound into which prenyl was deduced to have been introduced at position 8 or 3' were detected (Fig. 4). These compounds had not been prepared via the heterologous expression system with the use of a plant-derived gene in the past. 6-Geranylnaringenin (peak c in Fig. 4) was compared with a standard sample (provided by Associate professor Tomohisa Kuzuyama, the University of Tokyo), and the same retention time and identical fragments were observed (Fig. 5). Thus, novel prenyltransferase (i.e., H1PT1 full-length: 1,224 bp, ORF: 308 a.a.; H1PT2 full-length: 1,233 bp, ORF: 411 a.a.) was obtained. The amino acid sequences of proteins encoded by H1PT1 and H1PT2 are shown in SEQ ID NOs: 1 and 2, respectively. The nucleotide sequences of H1PT1 and H1PT2 are shown in SEQ ID NOs: 3 and 4, respectively. As is apparent from the results shown in Fig. 3, H1PT1 activity and H1PT2 activity similarly resulted in the generation of a plurality of products, and H1PT1 activity was higher. Thus, H1PT1 was mainly subjected to analysis as described below; however, it was also possible to analyze H1PT2 in the same manner.

### Example 4: Analysis of substrate specificity of recombinant H1PT1 using yeast expression system

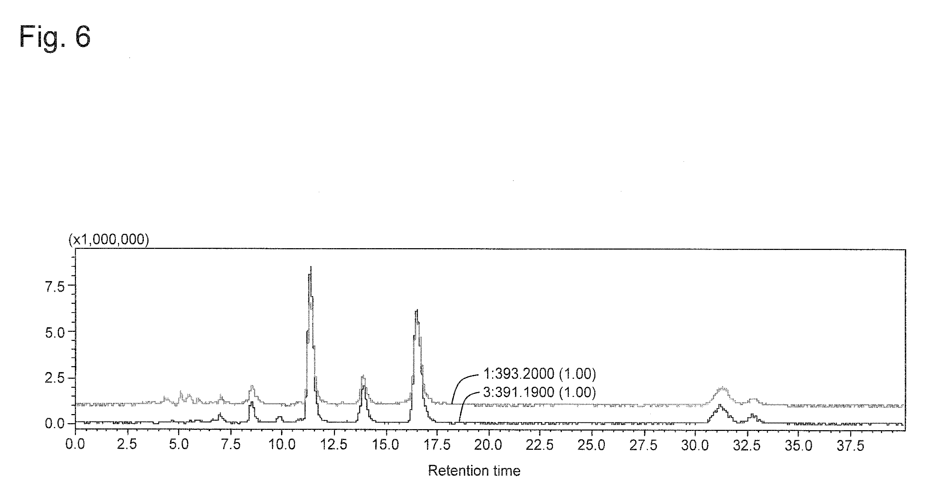

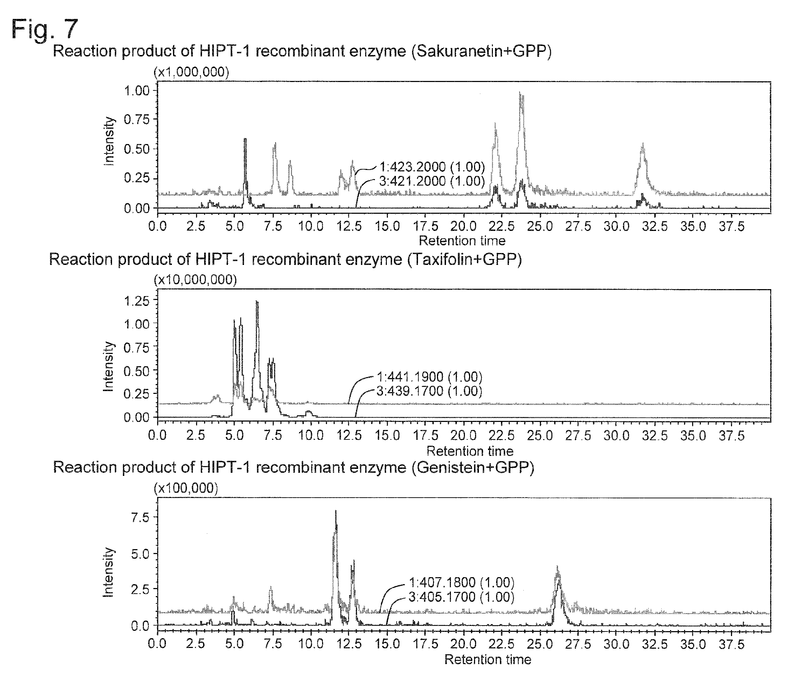

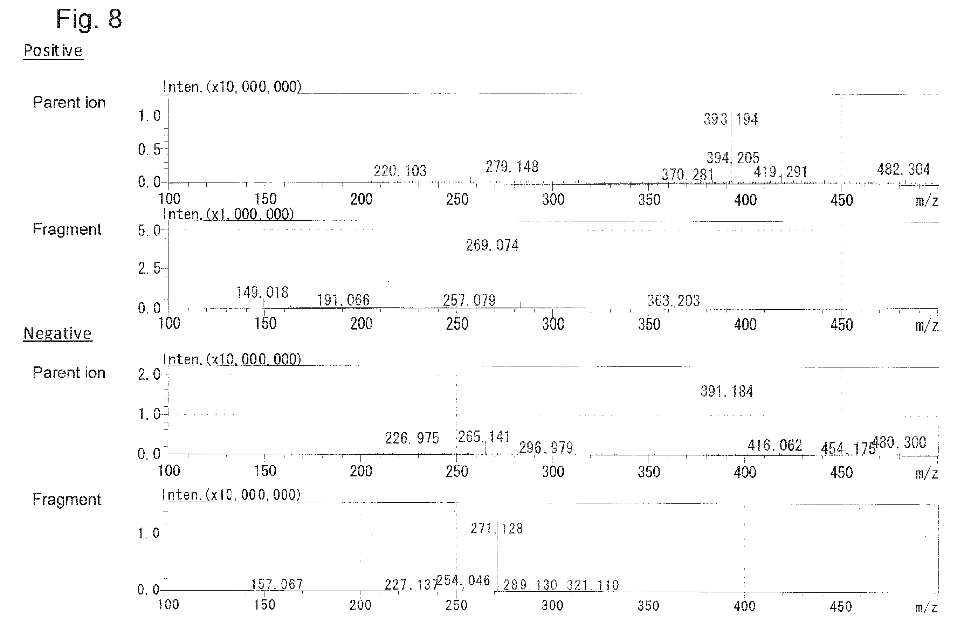

The *Sophora flavescens* gene (JP Patent Publication (Kokai) No. 2008-220304 A) and the soybean gene (Akashi et al., Plant Physiology 149: 683-693, 2009), which are already known, are known to encode enzymes that interact with limited substrates with very high specificity. The enzymatic properties of recombinant H1PT1 were analyzed using LCMS-IT-TOF. Geranyl diphosphate was used as a prenyl group donor for prenylation of various flavonoid-associated compounds (e.g., isoliquiritigenin, sakuranetin, taxifolin, or genistein). Surprisingly, activity of transferring a prenyl group to all substrates was observed (Figs. 6 and 7). In particular, xanthoangelol resulting from transfer of a prenyl group to isoliquiritigenin was compared with a standard sample (provided by associate professor Tomohisa Kuzuyama, the University of Tokyo) and found to exhibit the same retention time and have the same fragments (Fig. 8).

### Example 5: Genome nucleotide sequences of H1PT1 and H1PT2, and comparison with other varieties

Hop plants contain resin components in every part of the body. Such resin components were found to serve as contaminants and inhibit various reactions when conducting molecular biological analysis, such as PCR. Thus, DNAs were extracted from "Kirin II" and "Toyomidori" by the Nucleon PhytoPure method (GE Healthcare Biosciences). Two primers (ATGGAGCTCTCTTCAGTTTCTAGC (SEQ ID NO: 5): U866; TCCTTTTGCTGTGTATGGTCTT (SEQ ID NO: 6): U855) were prepared based on the nucleotide sequence as shown in SEQ ID NO: 4, and a hop genomic gene fragment (about 1.2 kb) was amplified via PCR. The amplified fragment was subjected to direct nucleotide sequencing and cloning of a part of the sequence into E. coli with the use of a TOPOTA cloning kit (Invitrogen) to determine the genome nucleotide sequence of the region of interest. The presence of an intron between G 282 and G 283 of the cDNA of "Kirin II" (between G 276 and G 277 in the case of "Toyomidori") was observed. The genome nucleotide sequences of the both varieties were compared. As a result, a residue 16 of the intron was found to be T in the case of "Kirin II" and A in the case of "Toyomidori." Hop varieties can be easily distinguished by the method of detecting single nucleotide polymorphisms. In addition, detection of single nucleotide polymorphisms enables monitoring of breeding processes, variety evaluation and genetic analysis such as QTL by analyzing the correlation between the polymorphisms and the amount of prenylated compounds or their composition. Thus, gene markers can be obtained.

As described above, the presence of a large number of polymorphisms in an intron is deduced. Thus, the intron is considered to be a region at which nucleotide sequence differences can be easily detected. The genome sequence of "Kirin II" corresponding to SEQ ID NO: 3 is shown in SEQ ID NO: 11. As is apparent from SEQ ID NO: 11, H1PT1 in "Kirin II" has 6 introns. Based on such nucleotide sequence information, a primer that amplifies genomic DNA can be designed for use at an arbitrary site of the gene.

### Example 6: Gene expression analysis in hop varieties or lineages

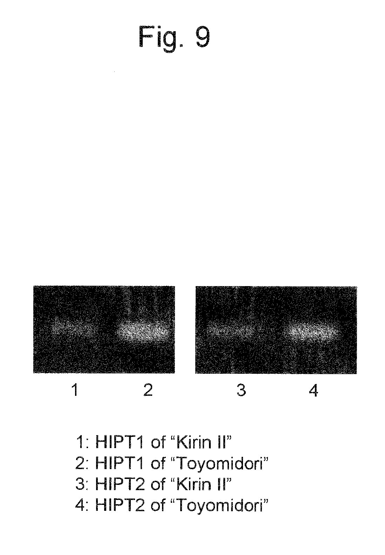

Total RNAs were extracted from leaves of hop varieties ("Kirin II" and "Toyomidori") using CTAB. cDNA was synthesized using the SuperScript III First-Strand Synthesis System (Invitrogen). Primers were prepared based on the nucleotide sequences shown in SEQ ID NOs: 3 and 4 (GCCCATTCCATTTGTAGCAG (SEQ ID NO: 7): U862 and GCCCCAATCACAAGATAACAA (SEQ ID NO: 8): U849 for H1PT1; TGTATGTTGGGAGTATGTAAGACC (SEQ ID NO: 9): U864 and GCTGTAATGGGATTCTTCTTCC (SEQ ID NO: 10): U851 for H1PT2), and RT-PCR was carried out. The "Toyomidori" variety is known to contain larger quantities of prenylated compounds than the "Kirin II" variety (the average α acid content of crops produced in 2001 to 2002 in Japan, which represents the total quantity of humulones, was 5.9% in the case of "Kirin II" and 11.9% in the case of "Toyomidori"). As shown in Fig. 9, expression levels of both H1PT1 and H1PT2 genes were found to be higher in "Toyomidori" than in "Kirin II." Thus, detection of the expression level can be carried out with the use of leaves in addition to the cones, and analysis of the correlation between the expression level and the amount of prenylated compounds or their composition enables variety evaluation and genetic analysis such as QTL. Thus gene expression markers can be obtained.

### Industrial Applicability

The method for producing and detecting an organism using a novel prenyltransferase and a gene thereof according to the present invention is useful for development of a technique for production of prenylated compounds using organisms such as plants or selection of hop varieties.

### Sequence Listing Free Text

### SEQ ID NOs: 5 to 10; primers

All publications, patents, and patent applications cited herein are incorporated herein by reference in their entirety.

## Claims

1. A protein (a) or (b) below:
(a) a protein comprising the amino acid sequence as shown in SEQ ID NO: 1; or
(b) a protein comprising an amino acid sequence derived from the amino acid sequence as shown in SEQ ID NO: 1 by deletion, substitution, insertion, or addition of 1 or several amino acids and having prenyltransferase activity.

2. A gene comprising any of DNAs (c) to (f) below:
(c) DNA comprising the nucleotide sequence as shown in SEQ ID NO: 3;
(d) DNA hybridizing under stringent conditions to DNA comprising a nucleotide sequence complementary to DNA comprising the nucleotide sequence as shown in SEQ ID NO: 3 and encoding a protein having prenyltransferase activity;
(e) DNA comprising a nucleotide sequence having 80% or higher sequence identity with the nucleotide sequence as shown in SEQ ID NO: 3 and encoding a protein having prenyltransferase activity; and
(f) DNA comprising a degenerate variant of the nucleotide sequence as shown in SEQ ID NO: 3.

3. A protein (g) or (h) below:
(g) a protein comprising the amino acid sequence as shown in SEQ ID NO: 2; or
(h) a protein comprising an amino acid sequence derived from the amino acid sequence as shown in SEQ ID NO: 2 by deletion, substitution, insertion, or addition of 1 or several amino acids and having prenyltransferase activity.

4. A gene comprising any of DNA (i) to (1) below:
(i) DNA comprising the nucleotide sequence as shown in SEQ ID NO: 4;
(j) DNA hybridizing under stringent conditions to DNA comprising a nucleotide sequence complementary to DNA comprising the nucleotide sequence as shown in SEQ ID NO: 4 and encoding a protein having prenyltransferase activity;
(k) DNA comprising a nucleotide sequence having 80% or higher homology to the nucleotide sequence as shown in SEQ ID NO: 4 and encoding a protein having prenyltransferase activity; and
(1) DNA comprising a degenerate variant of the nucleotide sequence as shown in SEQ ID NO: 4.

5. A recombinant vector comprising the gene according to claim 2 or 4.

6. A transformant into which the recombinant vector according to claim 5 has been introduced.

7. The transformant according to claim 6, which is a plant.

8. A method for detecting the presence of mutation and/or polymorphism of a gene encoding prenyltransferase in a plant comprising steps of:
(i) isolating a nucleic acid, which is genomic DNA or RNA, from a plant;
(ii) synthesizing cDNA via reverse transcription when the nucleic acid of (i) is RNA;
(iii) amplifying a gene fragment comprising the nucleotide sequence as shown in SEQ ID NO: 3, SEQ ID NO: 4, or SEQ ID NO: 11 from DNA obtained by step (i) or (ii); and
(iv) determining the presence of mutation and/or polymorphism in DNA.

9. The method according to claim 8, wherein the plant is of the genus *Humulus.*

10. A method for selecting a plant comprising mutation and/or polymorphism by detecting mutation and/or polymorphism of a gene encoding a prenyltransferase by the method according to claim 8 or 9.

11. A plant selected by the method according to claim 9 having mutation and/or polymorphism of a gene encoding a prenyltransferase.

12. The plant according to claim 11, which is of the genus *Humulus.*

13. The method for selecting a plant according to claim 8 or 9 comprising selecting a plant having the capacity for expressing a gene encoding a prenyltransferase or activity of a prenyltransferase to be encoded that is altered from that of an existing plant variety.

14. A plant selected by the method according to claim 13 having the capacity for expressing a gene encoding a prenyltransferase or activity of a prenyltransferase that is altered from that of an existing plant variety.

15. The plant according to claim 14, which is of the genus *Humulus.*


SEQUENCE LISTING

<110>  KIRIN HOLDINGS KABUSHIKI KAISHA

<120>  A novel protein having the plenyltransferase activity and a gene
       encoding the same

<130>  PH-4418-PCT

<150>  JP 2009-146754
<151>  2009-06-19

<160>  11

<170>  PatentIn version 3.4

<210>  1
<211>  408
<212>  PRT
<213>  Humulus lupulus

<400>  1

Met Glu Leu Ser Ser Ala Cys Asn Leu Ser Leu Lys Pro Asn Tyr Tyr
1               5                   10                  15

Tyr Tyr Pro Thr Ser Leu Phe Pro Ser Asn Asn Ser Tyr Asn Asn Leu
            20                  25                  30

Lys Ala Ser Ser Tyr Tyr Gln Thr Gln Arg Pro Ile Lys Cys Cys Ser
        35                  40                  45

Tyr Ser Pro Ser Lys Tyr Cys Ser Thr Lys Lys Leu Gln Thr Thr His
    50                  55                  60

Leu Leu Gly Leu Tyr Ala Lys His Lys Cys Leu Lys Pro Phe Ser Ile
65                  70                  75                  80

Gly His Leu Pro Arg Pro Asn Ser Leu Thr Ala Trp Ser His Gln Ser
                85                  90                  95

Glu Phe Pro Ser Thr Ile Val Thr Lys Gly Ser Asn Phe Gly His Ala
            100                 105                 110

Ser Trp Lys Phe Val Arg Pro Ile Pro Phe Val Ala Val Ser Ile Ile
        115                 120                 125

Cys Thr Ser Leu Phe Gly Ala Glu Leu Leu Lys Asn Pro Asn Leu Phe
    130                 135                 140

Ser Trp Gln Leu Met Phe Asp Ala Phe Gln Gly Leu Val Val Ile Leu
145                 150                 155                 160

Leu Tyr His Ile Tyr Ile Asn Gly Leu Asn Gln Ile Tyr Asp Leu Glu
                165                 170                 175

Ser Asp Arg Ile Asn Lys Pro Asp Leu Pro Leu Ala Ala Glu Glu Met
            180                 185                 190

Ser Val Lys Ser Ala Trp Phe Leu Thr Ile Phe Ser Ala Val Ala Ser
        195                 200                 205

Leu Leu Leu Met Ile Lys Leu Lys Cys Gly Leu Phe Leu Thr Cys Met
    210                 215                 220

Tyr Cys Cys Tyr Leu Val Ile Gly Ala Met Tyr Ser Val Pro Pro Phe
225                 230                 235                 240

Arg Trp Lys Met Asn Thr Phe Thr Ser Thr Leu Trp Asn Phe Ser Glu
                245                 250                 255

Ile Gly Ile Gly Ile Asn Phe Leu Ile Asn Tyr Ala Ser Arg Ala Thr
            260                 265                 270

Leu Gly Leu Pro Phe Gln Trp Arg Pro Pro Phe Thr Phe Ile Ile Gly
        275                 280                 285

Phe Val Ser Thr Leu Ser Ile Ile Leu Ser Ile Leu Lys Asp Val Pro
    290                 295                 300

Asp Val Glu Gly Asp Lys Lys Val Gly Met Ser Thr Leu Pro Val Ile
305                 310                 315                 320

Phe Gly Ala Arg Thr Ile Val Leu Val Gly Ser Gly Phe Phe Leu Leu
                325                 330                 335

Asn Tyr Val Ala Ala Ile Gly Val Ala Ile Met Trp Pro Gln Ala Phe
            340                 345                 350

Lys Gly Tyr Ile Met Ile Pro Ala His Ala Ile Phe Ala Ser Ala Leu
        355                 360                 365

Ile Phe Lys Thr Trp Leu Leu Asp Lys Ala Asn Tyr Ala Lys Glu Ala
    370                 375                 380

Ser Asp Ser Tyr Tyr His Phe Leu Trp Phe Leu Met Ile Ala Glu Tyr
385                 390                 395                 400

Ile Leu Tyr Pro Phe Ile Ser Thr
                405

<210>  2
<211>  411
<212>  PRT

<213>  Humulus lupulus

<400>  2

Met Glu Leu Ser Ser Val Ser Ser Phe Ser Leu Gly Thr Asn Pro Phe
1               5                   10                  15

Ile Ser Ile Pro His Asn Asn Asn Asn Leu Lys Val Ser Ser Tyr Cys
            20                  25                  30

Cys Lys Ser Lys Ser Arg Val Ile Asn Ser Thr Asn Ser Lys His Cys
        35                  40                  45

Ser Pro Asn Asn Asn Asn Asn Thr Ser Asn Lys Thr Thr His Leu Leu
    50                  55                  60

Gly Leu Tyr Gly Gln Ser Arg Cys Leu Leu Lys Pro Leu Ser Phe Ile
65                  70                  75                  80

Ser Cys Asn Asp Gln Arg Gly Asn Ser Ile Arg Ala Ser Ala Gln Ile
                85                  90                  95

Glu Asp Arg Pro Pro Glu Ser Gly Asn Leu Ser Ala Leu Thr Asn Val
            100                 105                 110

Lys Asp Phe Val Ser Val Cys Trp Glu Tyr Val Arg Pro Tyr Thr Ala
        115                 120                 125

Lys Gly Val Ile Ile Cys Ser Ser Cys Leu Phe Gly Arg Glu Leu Leu
    130                 135                 140

Glu Asn Pro Asn Leu Phe Ser Arg Pro Leu Ile Phe Arg Ala Leu Leu
145                 150                 155                 160

Gly Met Leu Ala Ile Leu Gly Ser Cys Phe Tyr Thr Ala Gly Ile Asn
                165                 170                 175

Gln Ile Phe Asp Met Asp Ile Asp Arg Ile Asn Lys Pro Asp Leu Pro
            180                 185                 190

Leu Val Ser Gly Arg Ile Ser Val Glu Ser Ala Trp Leu Leu Thr Leu
        195                 200                 205

Ser Pro Ala Ile Ile Gly Phe Ile Leu Ile Leu Lys Leu Asn Ser Gly
    210                 215                 220

Pro Leu Leu Thr Ser Leu Tyr Cys Leu Ala Ile Leu Ser Gly Thr Ile
225                 230                 235                 240

Tyr Ser Val Pro Pro Phe Arg Trp Lys Lys Asn Pro Ile Thr Ala Phe

245                 250                 255

Leu Cys Ile Leu Met Ile His Ala Gly Leu Asn Phe Ser Val Tyr Tyr
            260                 265                 270

Ala Ser Arg Ala Ala Leu Gly Leu Ala Phe Ala Trp Ser Pro Ser Phe
        275                 280                 285

Ser Phe Ile Thr Ala Phe Ile Thr Phe Met Thr Leu Thr Leu Ala Ser
    290                 295                 300

Ser Lys Asp Leu Ser Asp Ile Asn Gly Asp Arg Lys Phe Gly Val Glu
305                 310                 315                 320

Thr Phe Ala Thr Lys Leu Gly Ala Lys Asn Ile Thr Leu Leu Gly Thr
                325                 330                 335

Gly Leu Leu Leu Leu Asn Tyr Val Ala Ala Ile Ser Thr Ala Ile Ile
            340                 345                 350

Trp Pro Lys Ala Phe Lys Ser Asn Ile Met Leu Leu Ser His Ala Ile
        355                 360                 365

Leu Ala Phe Ser Leu Ile Phe Gln Ala Arg Glu Leu Asp Arg Thr Asn
    370                 375                 380

Tyr Thr Pro Glu Ala Cys Lys Ser Phe Tyr Glu Phe Ile Trp Ile Leu
385                 390                 395                 400

Phe Ser Ala Glu Tyr Val Val Tyr Leu Phe Ile
                405                 410

<210>  3
<211>  1224
<212>  DNA
<213>  Humulus lupulus

<400>  3
atggagctct catcagcttg taatctttca cttaaaccta attattatta ttatccaact      60

tcattattcc caagtaataa tagttataat aatctcaagg cctcatctta ttaccaaaca     120

cagagaccta tcaaatgttg tagttattct ccttcaaaat attgctccac caagaagttg     180

cagacaacac atctacttgg gctctatgca aaacacaaat gcttgaaacc tttttcaatc     240

ggtcatcttc caaggccaaa ttcccttacg gcttggtctc atcaaagtga atttcctagt     300

acaatagtta ccaaaggttc aaattttgga catgcttcgt ggaaattcgt aaggcccatt     360

ccatttgtag cagtatctat catatgcact tctttgtttg gagcagagct gttaaagaac     420

ccaaatctat ttagctggca attgatgttt gatgcattcc aaggcttagt ggttatatta     480

ctataccata tttacataaa tggcctcaat cagatttacg atttggaaag tgacaggata 540

aacaaaccag atttaccact agctgcagag gaaatgtcag ttaaatcagc ttggttcttg 600

acaatattta gtgcagtagc cagcttacta ttgatgatta agttgaagtg tggactattc 660

cttacttgta tgtactgttg ttatcttgtg attggggcta tgtattctgt tcctcccttt 720

agatggaaga tgaatacttt tacatcaact ctctggaact tttcggaaat aggtatcggt 780

ataaattttc tgatcaacta tgctagcaga gctactcttg gacttccatt tcagtggagg 840

cctccattta ctttcatcat tggctttgtt tcaactttaa gtattatctt gagcatccta 900

aaagatgttc ctgatgtaga aggtgacaag aaggttggca tgtcaacgtt accagtaata 960

tttggtgcta gaaccatagt attagttggt tctggatttt ttctcctaaa ttatgtagct 1020

gctattggtg ttgccattat gtggcctcag gctttcaagg gttacataat gattcctgct 1080

catgcaatct ttgcatctgc cttaatcttc aagacttggt tgttggacaa agcaaattac 1140

gctaaggagg ccagtgacag ctactatcac ttcctgtggt ttctaatgat tgcggaatac 1200

attttgtatc ctttcatctc tacc 1224

<210> 4
<211> 1233
<212> DNA
<213> Humulus lupulus

<400> 4
atggagctct cttcagtttc tagcttttca cttggaacta atccatttat atcaatcccc 60

cataataata ataatctcaa ggtctcatct tactgttgta aaagcaagag cagagtaatc 120

aattccacaa actcaaagca ttgttccccc aacaacaaca acaacacctc taacaagaca 180

acacatcttc ttgggttgta cggacagagc agatgcttat taaaaccttt atcatttatc 240

agctgcaacg accaaagggg aaattcaatt agggcttctg cacaaattga agatcgacct 300

cctgaatctg gaaatctttc ggcacttaca aatgttaaag actttgtaag tgtatgttgg 360

gagtatgtaa gaccatacac agcaaaagga gttattatat gctctagttg tttatttgga 420

agagaattgt tggagaaccc aaatctattt agtaggcctc taatttttag ggcactcttg 480

ggaatgttgg ctatactggg ctcttgtttt tatacagctg gcatcaatca aatttttgat 540

atggatattg acaggataaa caaaccagat ttaccactgg tttcagggcg tatttctgtg 600

gaatcagctt ggttattgac gttaagtcct gcaataattg gcttcatatt gattcttaaa 660

ttgaactcag gaccactcct tacttctcta tactgtttgg ccattttgag tgggactatc 720

tattctgttc ctccatttag atggaagaag aatcccatta cagcatttct ttgtattctt 780

atgattcatg caggtttaaa cttttctgta tattatgcct ctagagcagc acttggactt 840

gcatttgcat ggagcccttc attttccttc atcactgcct ttattacatt tatgacgcta 900

acgttggctt cctccaaaga tctttctgac ataaatggag atcgcaagtt tggtgttgaa 960

acctttgcaa ccaagcttgg tgcaaaaaac attacattac ttggcacagg acttctcctc 1020

ctaaactatg tagcagctat atctactgcc attatatggc ctaaggcttt caagagtaac 1080

ataatgctgc tttctcatgc aatcttagca ttttccttaa tcttccaggc tcgagagttg 1140

gatcgaacga actacactcc ggaagcgtgc aaaagcttct atgaattcat ctggatcctc 1200

ttctctgcgg aatacgttgt atatctgttc att 1233

<210> 5
<211> 24
<212> DNA
<213> Artificial

<220>
<223> primer

<400> 5
atggagctct cttcagtttc tagc 24

<210> 6
<211> 22
<212> DNA
<213> Artificial

<220>
<223> primer

<400> 6
tccttttgct gtgtatggtc tt 22

<210> 7
<211> 20
<212> DNA
<213> Artificial

<220>
<223> primer

<400> 7
gcccattcca tttgtagcag 20

<210> 8
<211> 21
<212> DNA
<213> Artificial

<220>
<223> primer

<400> 8
gccccaatca caagataaca a 21

<210> 9
<211> 24
<212> DNA
<213> Artificial

<220>

<223>  primer

<400>  9
tgtatgttgg gagtatgtaa gacc                                              24


<210>  10
<211>  22
<212>  DNA
<213>  Artificial

<220>
<223>  primer

<400>  10
gctgtaatgg gattcttctt cc                                                22


<210>  11
<211>  4897
<212>  DNA
<213>  Humulus lupulus

<400>  11
   gttattctct ctctttccag ttgagatgga gctctcatca gcttgtaatc tttcacttaa      60
   acctaattat tattattatg ccaacttcat tattcccaag taataatagt tataataatc     120
   tcaaggcctc atcttattac caaacacaga gacctatcaa atgttgtagt tattctcctt     180
   caaaatattg ctccaccaag aagttgcaga caacacatct acttgggctc tatgcaaaac     240
   acaaatgctt gaaacctttt tcaatcggtc atcttccaag gccaaattcc cttacggctt     300
   ggtctcatca aagtgaattt cctagtacaa tagttaccaa aggttcaaat tttggacatg     360
   cttcgtggaa attcgtaagg cccattccat ttgtagcagt atctatcata tgcacttctt     420
   tgtttggagc agagctgtta aagaacccaa atctatttag ctggcaattg atgtttgatg     480
   cattccaagg cttagtggtt atattactat cccatattta cataaatggc ctcaatcaga     540
   tttacgattt ggaaagtgac aggataaaca aaccagattt accactagct gcagaggaaa     600
   tgtcagttaa atcagcttgg ttcttgacaa tatttagtgc agtagccagc ttactattga     660
   tgattaagtt gaagtgtgga ctattcctta cttgtatgta ctgttgttat cttgtgattg     720
   gggctatgta ttctgttcct ccctttagat ggaagatgaa tacttttaca tcaactctct     780
   ggaacttttc ggtatgaaat gttttacttt ttcatttaca gacattataa cacttatact     840
   ttagcaccct ttaatgggat tgatgtcata ataaattaaa atatgtcatg tatcaataat     900
   gttagacact ttaagatctc ttataccaat gctcattcat ttatcattaa ccccaactaa     960
   ttaattgtgt aattctactg cttaatctta tatatattaa tatatctagt taccatactg    1020
   tattttttta ttaaaaaaaa gttatattat ctatttctta tcatatactg aaaaaaataa    1080
   cagaaaaaaa tttatataat acacaggaaa taggtatcgg tataaatttt ctgatcaact    1140
   atgctagcag agctactctt ggacttccat ttcagtggag gtaggttcca aattttcata    1200
   acatacctca agtaaaggaa tgcaaaacaa acactctcta tcttatcttg tatgccacat    1260
   tttttcgaat aattgagaat tttaatatat tagattatat ttttgaagtt attttatttt 1320
   taaaatattt cttcttgaat tattgacatt atatagtttt acctttattt aacatgacat 1380
   gtcatcacca ttgtcgctaa gcatgccatg taaccattcc atcttttttt tttttgaagg 1440
   agaattcctt ttgtcctttc gtacatatat ttaaatggta aaactttacc gtaacattta 1500
   accaaaatta tttttattat cttatatatt tataaaaaaa tatgtattta acgatttttg 1560
   tttgttttaa taacccaaaa attgaacaaa atatactttt aaaatcaatt aaataaatat 1620
   ttattgatta tattaataaa ttttttaaat attataataa tatttaatac aaaactagat 1680
   atttaataat tatatcaata taaattcaaa tgttataaaa tatcattatt ataagggtca 1740
   tttggcaaaa tgatctattt tttgaagtca ttttgtactc taacctagtt ttgataattt 1800
   atgatgtcga aaacaaagtt caaaatagtt agttgcacac atataaaaaa tgtactcgtg 1860
   caactaatta tttgaactat atttttaaca cagtacatta ttttggaatt tcctgaaatc 1920
   taaacagata tctattataa cttcaatgta tactgtcacg taaaagaatt tggattatag 1980
   ttttttcaca tggtaaaaat acatttgtcc ccacctgtag agacaaaaat ggtatctcta 2040
   cctaaaaaag tactatatat atattaatat atatatggat acatgaaagc tcaaaaaaaa 2100
   aaaattaatg tcctatgaaa ttaattttta taacaatatc taataaatat ttatatatat 2160
   aaatttgact caggcctcca tttactttca tcattggctt tgtttcaact ttaagtatta 2220
   tcttgagcat cctaaaagat gttcctgatg tagaaggtga caagaagtaa gttttttttt 2280
   tccctttctt ttttaattgt atgcgattat aaacagttct aactaattaa ataaaaaaac 2340
   gcaatcatat ataaacaaaa ataatattat catcttaatt ttcagggttg gcatgtcaac 2400
   gttaccagta atatttggtg ctagaaccat agtattagtt ggttctggat tttttctcct 2460
   aaattatgta gctgctattg gtgttgccat tatgtggcct caggtactca ccactaaaaa 2520
   atcaaattag caatttttta gttgtatcat aaaatctttt gtaattaaat gtcatttata 2580
   ttcgtgataa aaaattattt tgtcactaaa aaaagtaata ttcaatcaca aatttatttt 2640
   gtcacactta aatttttgta actaatattt ttttagccac aaaaacattt tagtaataat 2700
   agatatatgt taatattttt tatcacaatt tttgttttgt ttttaatcac aataactttg 2760
   ttgtaattaa aggtagaaaa atgatgatcg atcttataaa atagtaggaa caatattaat 2820
   aatatatacc ttcaaaacct tagtcttttc aatttcttat ctacacaggc tttcaagggt 2880
   tacataatga ttcctgctca tgcaatcttt gcatctgcct taatcttcaa ggtttgtcat 2940
   ataaatcaca actaatatat ttctttatat aaaaaaatat attgagaacg aaattttttt 3000
   attttaacga tttttttgtt aactttaata taatattgta aatatttaat ggaatataca 3060
   tttaaaatta attaaaaata aatatttatt aattgtatta atataaattt aaatattata 3120
   aaatattatt attataataa tatttaatat aagactacaa atttaataat tatattaata 3180
   taaatataaa tttaaatgtt ataaaatatc attattataa taatatataa tacaaagtta 3240
   gatatttaat aattatatta atataaattc aaatattata aaatattatt atgataataa 3300
   tatataatct taattttttt actaattata ttagtatgaa tttaaatatt ataaaatatt 3360
   gttattataa caatatttaa tacaagacta gatatttaat aattatatta atataaattt 3420
   aaatattata aatacataat tttaattttt attaaaaaaa atatcatttc tatttaaaaa 3480
   aaaaagaaaa catattattt tttaattgat ctaacttata taaatatata ttcatattga 3540
   ataataataa atattataaa tatattatat atatgtatcg tttgtaaata aatagtataa 3600
   ctgtgtaact acataagaaa ttaaaataac atttattttc tatcaagaat aaacaagatt 3660
   attctccaat cattaaggtg taatttgaat aatatcatta atgttttgta ttataaataa 3720
   agtatttgtg atataacaag ttatcatatt atatatattt aaaaaaaaaa cataaacaat 3780
   gttttggttt aattttttta atatatttat gtaattattt tataaataat attgtttatt 3840
   tatttgaaat ttacatgata attatataaa tttaataaaa ataattaata aattttataa 3900
   ataaactaac cgaacgtgca tggtatgttt cttgtgtcta gtatatatat atatataggg 3960
   atgaatataa cttcgatata atttcatttt taaattattt caataatctt aaattattta 4020
   gagtatgcaa cggaatcgat cttagtatat tcttattagc atctccaatg aaatgttaaa 4080
   ttggtgatgc attgctaaaa tatacacttt gcaaaaaagt tactccaata gtgtaccaaa 4140
   agttgtgcca aatttggcat acgctaaaag ttggaccaaa tttgacacac aatataacat 4200
   gatgtatatt ttaaatcacc ataaatgcta cactttttta tttaatttta tgcaatattt 4260
   tattattgtt tttatctatc actattggaa aacccttttc tatttcaatt taccaaccaa 4320
   ataatgaaat gaaataaaaa agcatttcat tcatttccct taaaccaaac attattattt 4380
   tattattatc tttaactatc aagaataaaa tagtaaaatt attacttttg gtatattttc 4440
   aacaaatatc aaataaacaa taataaaata ttaatttttg cataaacttt tacaataatt 4500
   ttacattgta gcgtaatact aaaatttgtt tcgtattact cattgatttt tttgtatcaa 4560
   atatatcaca ttggagagat ggtcttactt atttttattt gtgtgtgcat atagacttgg 4620
   ttgttggaca aagcaaatta cgctaaggta atagcaagat aatacaatat gaattgaact 4680
   ctctctctct ctctctctcg ctttttctct gattatttgt ttgggcaata catgtacagg 4740
   aggccagtga cagctactat cacttcctgt ggtttctaat gattgcggaa tacattttgt 4800
   atcctttcat ctctacctaa cctaatttca tttacttgca attcgcttac tgggcttgta 4860
   ctactttttc tttctattaa ttatgggtac caacatc 4897